# EUROPEAN PATENT APPLICATION

(11) **EP 4 825 319 A1**
(43) Date of publication of application: **21.10.2026**
(21) Application number: 25745374.6
(22) Date of filing: 24.01.2025
(51) Int. Cl.: A61B 5/346, A61B 5/00, G16H 50/30

(54) **METHOD, DEVICE AND PROGRAM FOR DETERMINING PATIENT SEVERITY FROM BIOMETRIC DATA ON BASIS OF DEEP LEARNING MODEL**

(30) Priority: 26.01.2024 KR 20240012482
(71) Applicant: Medical AI Co., Ltd., Seoul 06180 (KR)
(72) Inventor: KWON, Joonmyoung, Seoul 06180 (KR); SON, Jeongmin, Seoul 06180 (KR)
(74) Representative: Patentanwaltskanzlei Matschnig & Forsthuber OG
(86) International application number: PCT/KR2025/001409
(87) International publication number: WO 2025/159552

(57) **Abstract**

The present disclosure provides a method, a device, and a computer program for determining a severity level of a patient based on a deep learning model. According to an embodiment of the present disclosure, the method comprises acquiring physiological data of the patient, and determining the severity level of the patient by analyzing the physiological data based on a pre-trained first neural network model. The pre-trained first neural network model is trained based on training data to which labels corresponding to patient states and treatment methods classified according to patient severity levels are assigned.

## Description

### TECHNICAL FIELD

The present disclosure relates to deep learning technology in the medical field, and more specifically, to a device and a method for diagnosing a severity level of a patient by analyzing physiological data of the patient using artificial intelligence technology.

### BACKGROUND OF THE INVENTION

Electrocardiogram(hereinafter "ECG") data is data that records electrical activity of the heart over time and accurately reflects physiological states such as heart rate and heart rhythm. ECG data can be acquired non-invasively and is useful for monitoring a patient's state in real time based on high accessibility and reliability. In particular, ECG data can be conveniently acquired through portable devices or in-hospital equipment, making it highly practical for data collection for diagnosis and treatment.

In the medical environment, the need for technology that can support precise and rapid decision-making in the process of determining the severity level of a patient is increasingly growing. Conventional severity level assessment methods include a complex process that heavily relies on the experience of medical personnel or requires comprehensive analysis of multiple diagnostic data. Such a process may make effective response difficult in emergency situations or in environments with limited medical resources. Therefore, development of a system that can monitor the severity level of a patient in real time based on ECG data and quantitatively evaluate the same is required.

### SUMMARY OF THE INVENTION

### TECHNICAL PROBLEM

The present disclosure has been conceived in response to the above-described background, and an object thereof is to provide a method, a device, and a program for determining a severity level of a patient from physiological data based on a deep learning model.

However, the problems to be solved by the present disclosure are not limited to the aforementioned problems, and other unmentioned problems will be clearly understood from the following description.

### TECHNICAL SOLUTION

According to an embodiment of the present disclosure to achieve the aforementioned objects, a method for determining a severity level of a patient based on a deep learning model, performed by a computing device including at least one processor, comprises acquiring physiological data of the patient, and determining the severity level of the patient by analyzing the physiological data based on a pre-trained first neural network model. The pre-trained first neural network model is trained based on training data to which labels corresponding to patient states and treatment methods classified according to patient severity levels are assigned.

Alternatively, the labels corresponding to patient states and treatment methods classified according to patient severity levels are classified into a patient in a normal state, a patient receiving outpatient treatment, a patient hospitalized in a general ward, a patient hospitalized in an intensive care unit, and a patient within a preset time period before cardiac arrest.

Alternatively, the determining of the severity level of the patient comprises inputting the physiological data into the pre-trained first neural network model to extract feature information corresponding to a state of the patient, acquiring a first score corresponding to the severity level of the patient based on the extracted feature information, and determining the severity level of the patient based on the acquired first score.

Alternatively, the determining of the severity level of the patient based on the acquired first score comprises determining the severity level of the patient as a normal state when the acquired first score is less than a first value, determining the severity level of the patient as a severity level amenable to outpatient treatment when the acquired first score is equal to or greater than the first value and less than a second value, determining the severity level of the patient as a severity level requiring hospitalization when the acquired first score is equal to or greater than the second value and less than a third value, determining the severity level of the patient as a severity level requiring intensive care when the acquired first score is equal to or greater than the third value and less than a fourth value, and determining the severity level of the patient as a state within a preset time period before cardiac arrest when the acquired first score is equal to or greater than the fourth value.

Alternatively, the labels corresponding to patient states and treatment methods classified according to patient severity levels comprise a fifth value corresponding to the patient in a normal state, a sixth value corresponding to the patient receiving outpatient treatment, a seventh value corresponding to the patient hospitalized in a general ward, an eighth value corresponding to the patient hospitalized in an intensive care unit, and a ninth value corresponding to the patient within a preset time period before cardiac arrest.

Alternatively, the first through fourth values are set by adjusting the fifth through ninth values with the average value of first scores corresponding to the normal state of the patient determined based on physiological data acquired from the patient in the past.

Alternatively, the method comprises, by analyzing the physiological data based on a pre-trained second neural network model to acquire a second score for likelihood of a disease in the patient, determining the severity level of the patient according to the disease based on the first and second scores.

Alternatively, the determining of the severity level of the patient according to the disease comprises determining the severity level of the patient according to the disease by determining relevance between the disease and the severity level of the patient based on the first score, the second score, and disease type information of the patient.

Alternatively, the pre-trained first and second neural network models share a network extracting, from the physiological data, feature information corresponding to the state of the patient.

According to an embodiment of the present disclosure to achieve the aforementioned objects, a computing device for determining a severity level of a patient based on a deep learning model comprises a processor comprising at least one core, a memory storing program code executable by the processor, and a communication interface. The processor is configured to acquire physiological data of the patient, and determine the severity level of the patient by analyzing the physiological data based on a pre-trained first neural network model. The pre-trained first neural network model is trained based on training data to which labels corresponding to patient states and treatment methods classified according to patient severity levels are assigned.

According to an embodiment of the present disclosure to achieve the aforementioned objects, a computer program stored on a computer-readable storage medium, when executed by one or more processors, causes operations for determining a severity level of a patient based on a deep learning model to be performed. The operations comprise acquiring physiological data of the patient, and determining the severity level of the patient by analyzing the physiological data based on a pre-trained first neural network model. The pre-trained first neural network model is trained based on training data to which labels corresponding to patient states and treatment methods classified according to patient severity levels are assigned.

### ADVANTAGEOUS EFFECTS

The method for determining a severity level of a patient from physiological data based on a deep learning model according to an embodiment of the present disclosure provides a technology capable of effectively analyzing physiological data of a patient and rapidly and accurately assessing the severity level. As a result, the patient's state is monitored in real time, which assists in determining an appropriate treatment method according to the severity level.

Furthermore, by overcoming the limitations of conventional severity level assessment methods that rely on medical personnel's experience or qualitative judgment and by quantitatively analyzing physiological data with a deep learning model, more objective and consistent results are provided.

### BRIEF DESCRIPTION OF THE DRAWINGS

FIG. 1 is a block diagram of a computing device according to an embodiment of the present disclosure.
FIG. 2 is a flowchart of a method for determining a severity level of a patient based on a deep learning model according to an embodiment of the present disclosure.
FIG. 3 is an exemplary diagram illustrating training data used for training a first neural network model to determine a severity level according to an embodiment of the present disclosure.
FIG. 4 is a block diagram of a second neural network model according to an embodiment of the present disclosure.
FIG. 5 is an exemplary diagram of a first neural network model and a second neural network model according to an embodiment of the present disclosure.
FIG. 6 is a detailed configuration diagram of a computing device according to another embodiment of the present disclosure.

### DETAILED DESCRIPTION OF THE EMBODIMENTS

Hereinafter, the present disclosure describes embodiments in detail with reference to the accompanying drawings so that a person of ordinary skill in the art to which the present disclosure pertains can easily implement the same. The embodiments presented in the present disclosure are provided to enable a person of ordinary skill in the art to use or implement the content of the present disclosure. Accordingly, various modifications to the embodiments of the present disclosure will be apparent to a person of ordinary skill in the art. That is, the present disclosure may be embodied in many different forms and is not to be construed as limited to the embodiments set forth herein.

Throughout the specification of the present disclosure, the same or similar reference numerals refer to the same or similar components. Furthermore, for clarity of description of the present disclosure, reference numerals for parts unrelated to the description of the present disclosure in the drawings may be omitted.

The term "or" as used in the present disclosure is intended to mean an inclusive "or" rather than an exclusive "or". That is, unless otherwise specified or clear from the context, "X uses A or B" is to be understood to mean any of the natural inclusive permutations. For example, if "X uses A or B" is used in the present disclosure, unless otherwise specified or clear from the context, it may be interpreted as any of the cases where X uses A, X uses B, or X uses both A and B.

The term "and/or" as used in the present disclosure is to be understood to refer to and include all possible combinations of one or more of the associated listed concepts.

The terms "comprise" and/or "comprising" as used in the present disclosure are to be understood to mean the presence of a specific feature and/or component. However, the terms "comprise" and/or "comprising" are to be understood as not precluding the presence or addition of one or more other features, other components, and/or combinations thereof.

In the present disclosure, unless otherwise specified or clear from the context to indicate a singular form, generally the singular is to be interpreted to include "one or more".

The term "N-th (where N is a natural number)" as used in the present disclosure may be understood as an expression used to distinguish components of the present disclosure from each other according to a certain standard, such as a functional perspective, a structural perspective, or for convenience of explanation. For example, in the present disclosure, components that perform different functional roles may be distinguished as a first component or a second component. However, components that are substantially the same within the technical spirit of the present disclosure but need to be distinguished for convenience of explanation may also be distinguished as a first component or a second component.

The term "acquire" as used in the present disclosure may be understood as not only receiving data via a wired/wireless communication network from an external device or system, but also generating data in an on-device form.

Meanwhile, the term "module" or "unit" as used in the present disclosure may be understood as a term referring to an independent functional unit that processes computing resources, such as a computer-related entity, firmware, software, or a portion thereof, hardware or a portion thereof, or a combination of software and hardware. A "module" or a "unit" may be a unit configured as a single element, or a unit expressed as a combination or a set of a plurality of elements. For example, in a narrow sense, a "module" or a "unit" may refer to a hardware element of a computing device or a set thereof, an application program that performs a specific function of software, a processing procedure implemented by software execution, or a set of instructions for program execution. Furthermore, in a broad sense, a "module" or a "unit" may refer to a computing device itself that constitutes a system, or an application executed on the computing device. However, the above-mentioned concepts are only examples, and the concepts of "module" or "unit" may be variously defined within the scope understandable by a person of ordinary skill in the art based on the content of the present disclosure.

The term "model" as used in the present disclosure may be understood as an abstraction for a system implemented using mathematical concepts and language to solve a specific problem, a set of software units for solving a specific problem, or a processing procedure for solving a specific problem. For example, a neural network "model" may refer to an entire system implemented as a neural network that has a problem-solving ability through learning. The neural network may have problem-solving ability by optimizing parameters that connect nodes or neurons through learning. A neural network "model" may include a single neural network or a set of neural networks in which a plurality of neural networks are combined.

The term "data" as used in the present disclosure may include "images", signals, and the like. The term "image" as used in the present disclosure may refer to multi-dimensional data composed of discrete image elements. In other words, an "image" may be understood as a term referring to a digital representation of an object that can be seen by the human eye. For example, an "image" may refer to multi-dimensional data composed of elements corresponding to pixels in a 2D image. An "image" may refer to multi-dimensional data composed of elements corresponding to voxels in a 3D image.

The foregoing description of the terms is for the purpose of aiding the understanding of the present disclosure. Therefore, it is to be noted that if the foregoing terms are not explicitly stated as limiting the content of the present disclosure, they are not used in a sense that limits the technical spirit of the content of the present disclosure.

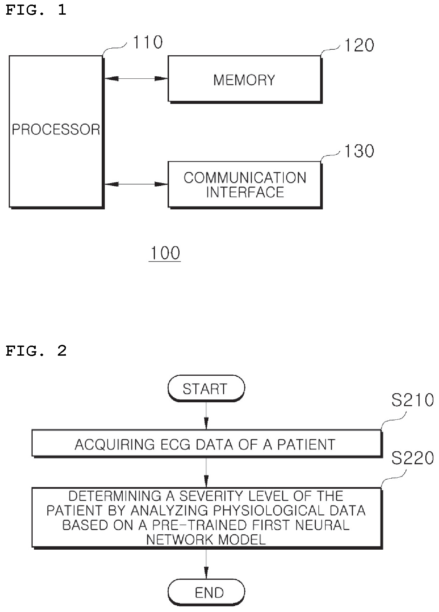

FIG. 1 is a block diagram of a computing device according to an embodiment of the present disclosure.

A computing device 100 according to an embodiment of the present disclosure may be a hardware device or a part of a hardware device that performs comprehensive processing and computation of data, or may be a software-based computing environment connected by a communication network. For example, the computing device 100 may be a server that performs intensive data processing functions and shares resources, or may be a client that shares resources via interaction with a server. Also, the computing device 100 may be a cloud system in which a plurality of servers and clients interact to comprehensively process data. The foregoing description is merely one example related to the type of the computing device 100, and thus the type of the computing device 100 may be variously configured within the scope understandable by a person of ordinary skill in the art based on the content of the present disclosure. Meanwhile, the computing device 100 may be implemented as various electronic devices such as a desktop, a notebook, a smartphone, a smart watch, a smart ring, and a server.

Referring to FIG. 1, the computing device 100 according to an embodiment of the present disclosure may include a processor 110, a memory 120, and a network unit 130. However, FIG. 1 is merely one example, and thus the computing device 100 may include other configurations for implementing a computing environment. Also, only some of the disclosed configurations may be included in the computing device 100.

The processor 110 according to an embodiment of the present disclosure may be understood as a component unit including hardware and/or software for performing computing operations. For example, the processor 110 may execute a computer program and perform data processing for machine learning. The processor 110 may process computational operations such as feature extraction for machine learning and error calculation based on backpropagation. The processor 110 for performing such data processing may include a central processing unit (CPU), a general-purpose graphics processing unit (GPGPU), a tensor processing unit (TPU), an application-specific integrated circuit (ASIC), or a field-programmable gate array (FPGA). The types of the processor 110 described above are only examples, and the type of the processor 110 may be variously configured within the scope understandable by a person of ordinary skill in the art based on the content of the present disclosure.

The processor 110 is connected to other components of the computing device 100 (i.e., the memory 120 and the communication interface 130) and controls overall operations of the computing device 100.

The memory 120 according to an embodiment of the present disclosure may be understood as a configuration unit including hardware and/or software for storing and managing data processed by the computing device 100. That is, the memory 120 may store data of any type generated or determined by the processor 110 and data of any type received by the communication interface 130. For example, the memory 120 may include at least one type of storage medium from among a flash memory type, a hard disk type, a multimedia card micro type, a card type memory, a random access memory (RAM), a static random access memory (SRAM), a read-only memory (ROM), an electrically erasable programmable read-only memory (EEPROM), a programmable read-only memory (PROM), a magnetic memory, a magnetic disk, and an optical disk. Also, the memory 120 may include a database system that controls and manages data in a predetermined system. The foregoing types of the memory 120 are merely examples, and thus the types of the memory 120 may be variously configured within the scope understandable by a person of ordinary skill in the art based on the content of the present disclosure.

The memory 120 may structure, organize, and manage data necessary for the processor 110 to perform operations, combinations thereof, and program code executable by the processor 110. For example, the memory 120 may store physiological data (aortic blood pressure data, electroencephalogram data, and ECG data) received through the communication interface 130, which is described below. Also, the memory 120 may store a neural network model trained to determine the severity level of a patient or a neural network model trained to determine reactivity to the likelihood of a disease in a patient, and may store program code for training each neural network model, program code for operating the neural network model to receive physiological data (aortic blood pressure data, electroencephalogram(hereinafter "EEG") data, ECG data, and the like) as input and to perform inference tailored to the purpose of the computing device 100, and processed data generated as the program code is executed.

The communication interface 130 according to an embodiment of the present disclosure may be understood as a component unit that transmits and receives data through any form of known wired/wireless communication system. For example, the communication interface 130 may perform data transmission and reception using a wired/wireless communication system such as a local area network (LAN), wideband code division multiple access (WCDMA), long term evolution (LTE), wireless broadband internet (WiBro), 5th generation mobile communication (5G), ultra wideband, ZigBee, radio frequency (RF) communication, wireless LAN, wireless fidelity (Wi-Fi), near field communication (NFC), or Bluetooth. The communication systems described above are only examples, and therefore, wired/wireless communication systems for data transmission and reception of the communication interface 130 may be variously applied in addition to the examples described above.

The communication interface 130 may receive data necessary for the processor 110 to perform operations via wired/wireless communication with any system or any client. In addition, the communication interface 130 may transmit data generated through the operations of the processor 110 via wired/wireless communication with any system or any client. For example, the communication interface 130 may receive physiological data of a patient via communication with a database within a hospital environment, a cloud server that performs tasks such as standardization of medical data, or a computing device. The communication interface 130 may transmit output data of the neural network model, intermediate data derived in the computation process of the processor 110, processed data, and the like via communication with the aforementioned database, server, or computing device.

FIG. 2 is a flowchart of a method for determining a severity level of a patient based on a deep learning model according to an embodiment of the present disclosure.

Referring to FIG. 2, the processor 110 acquires physiological data of a patient (S210). The physiological data may include ECG data. Specifically, the processor 110 may acquire ECG data of a patient acquired by an external physiological signal measurement device connected to the computing device 100. The processor 110 may receive the ECG data from the external physiological signal measurement device via the communication interface 130. Furthermore, the processor 110 may directly detect an electrical signal generated from the heart of the patient via a sensing unit of the computing device 100 to acquire the ECG data of the patient. To this end, the sensing unit includes one or more electrodes, and the processor 110 may measure the ECG of the patient as the one or more electrodes are attached to the body of the patient to acquire the ECG data from the patient. The processor 110 may acquire a single-lead ECG of the patient using a single electrode, or may acquire ECG data of various forms such as 3-lead, 6-lead, and 12-lead ECGs using a plurality of electrodes.

Meanwhile, without being limited thereto, the processor 110 may acquire various physiological data acquirable from the patient in addition to the ECG data. For example, the processor 110 may acquire various physiological data such as the patient's body temperature, photoplethysmography, heart rate, and EEG data. This may be determined based on the type of physiological data constituting the training data used for training the neural network model to identify the severity level of the patient. The sensing unit may include a temperature sensor, an image sensor, or an optical sensor to acquire various physiological data of the patient.

The processor 110 may analyze the acquired physiological data using a pre-trained neural network model and determine the severity level of the patient (S220). The severity level includes any information that quantifies a critical state of the patient. The severity level of the patient may be classified into a plurality of classes or grades. Hereinafter, for convenience of description of the present disclosure, a neural network model trained to determine the severity level of the patient is referred to as a first neural network model, and the physiological data is assumed to be ECG data.

The processor 110 may analyze the ECG data using the first neural network model trained based on training data including a plurality of ECG data. The processor 110 may extract feature information related to the severity level of the patient from the ECG data using the first neural network model and determine the severity level of the patient based on the extracted feature information. To this end, the pre-trained first neural network model may be trained based on training data to which labels corresponding to patient states and treatment methods classified according to patient severity levels are assigned.

That is, the patient states and treatment methods appropriate for each patient state may be classified according to severity levels, and training data to which labels relating to such patient states and treatment methods are assigned may be used to train the first neural network model. The labels include labels related to treatment methods and labels each indicating a state of a specific patient.

The processor 110 may acquire training data including input data composed of a plurality of ECG data acquired from different patients and label data to which labels indicating the severity level (or class) of each patient are assigned, and may pre-train the first neural network model using the acquired training data. The training data may include feature information (e.g., P-wave, QRS complex, T-wave) extracted from an ECG signal (more specifically, an ECG signal corresponding to the ECG data). The processor 110 may train the first neural network model to determine the severity level of the patient based on such feature information. The processor 110 may input the training data to the first neural network model and calculate a loss function based on a difference between an output value of the first neural network model in the training process and the label data. The loss function may be defined as a cross-entropy loss or an objective function that optimizes a balance between precision and recall. Based on the calculated loss function, the processor 110 may adjust weights of the neural network model through backpropagation. By repeating such a process, the processor 110 may improve the classification performance of the first neural network model with respect to the severity level, and may finally acquire the first neural network model trained to determine the severity level of the patient based on the ECG data.

Meanwhile, the ECG signals corresponding to the ECG data included in the training data used for training the first neural network model may commonly have a preset length. Correspondingly, when the ECG data is acquired from the patient, the processor 110 may divide the ECG signal corresponding to the acquired ECG data into a preset length or may divide the same into a plurality of segments by applying a window of a preset length along a time axis. For example, the ECG signal may be a signal measured with 12 leads having a length of 10 seconds and measured at 500 points per second.

The first neural network model may be implemented as a multi-layer perceptron (MLP), a convolutional neural network (CNN), a recurrent neural network (RNN), a generative adversarial network (GAN), or the like.

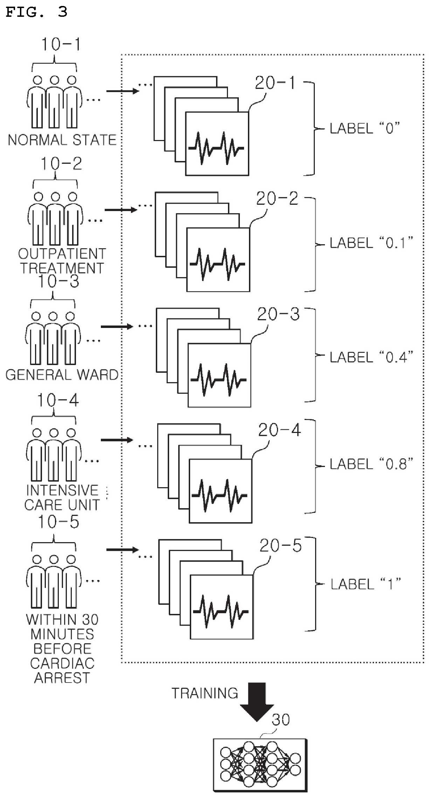

FIG. 3 is an exemplary diagram illustrating training data used for training a first neural network model to determine a severity level according to an embodiment of the present disclosure.

According to an embodiment of the present disclosure, the labels corresponding to patient states and treatment methods classified according to patient severity levels may be classified into: 1) a patient in a normal state, 2) a patient receiving outpatient treatment, 3) a patient hospitalized in a general ward, 4) a patient hospitalized in an intensive care unit, and 5) a patient within a preset time period before cardiac arrest. Specifically, referring to FIG. 3, the processor 110 may prepare input data composed of a plurality of ECG data 20-1 acquired from a plurality of patients 10-1 in a normal state, a plurality of ECG data 20-2 acquired from a plurality of patients 10-2 receiving outpatient treatment, a plurality of ECG data 20-3 acquired from a plurality of patients 10-3 hospitalized and receiving treatment in a general ward, a plurality of ECG data 20-4 acquired from a plurality of patients 40-1 hospitalized and receiving treatment in an intensive care unit, and a plurality of ECG data 20-5 acquired from a plurality of patients 10-5 within a preset time period (e.g., 30 minutes) before cardiac arrest. The processor 110 may prepare label data to which respective labels (i.e., a label for a patient in a normal state, a label for a patient receiving outpatient treatment, a label for a patient hospitalized in a general ward, a label for a patient hospitalized in an intensive care unit, and a label for a patient within a preset time period before cardiac arrest) corresponding to the input data are assigned. The processor 110 may train the first neural network model 30 with training data composed of the input data and the label data.

Meanwhile, according to an embodiment of the present disclosure, the labels corresponding to patient states and treatment methods classified according to patient severity levels may be set to specific values. The labels may include a fifth value corresponding to the patient in a normal state, a sixth value corresponding to the patient receiving outpatient treatment, a seventh value corresponding to the patient hospitalized in a general ward, an eighth value corresponding to the patient hospitalized in an intensive care unit, and a ninth value corresponding to the patient within a preset time period before cardiac arrest. For example, as illustrated in FIG. 3, a label of "0" may be assigned to the ECG acquired from the patient in a normal state, a label of "0.1" may be assigned to the ECG acquired from the patient receiving outpatient treatment, a label of "0.4" may be assigned to the ECG acquired from the patient receiving inpatient treatment in a general ward, a label of "0.8" may be assigned to the ECG acquired from the patient receiving treatment in an intensive care unit, and a label of "1" may be assigned to the ECG acquired from the patient within a preset time period before cardiac arrest. However, this is merely one example, and the specific values set as labels may be variously set.

According to an embodiment of the present disclosure, the processor 110 may input the acquired ECG data to the pre-trained first neural network model 30 to acquire a score relating to the severity level of the patient. As one example, the processor 110 may input the ECG data acquired from the patient to the pre-trained first neural network model 30 to acquire a score relating to the severity level of the patient. The score may be a probability value relating to the severity level output from the first neural network model 30. Hereinafter, a score acquired from the first neural network model 30 is referred to as a first score.

The processor 110 may determine the severity level of the patient as a normal state when the acquired first score is less than a first value, determine the severity level of the patient as a severity level amenable to outpatient treatment when the acquired first score is equal to or greater than the first value and less than a second value, determining the severity level of the patient as a severity level requiring hospitalization when the acquired first score is equal to or greater than the second value and less than a third value, determining the severity level of the patient as a severity level requiring intensive care when the acquired first score is equal to or greater than the third value and less than a fourth value, and determining the severity level of the patient as a state within a preset time period before cardiac arrest when the acquired first score is equal to or greater than the fourth value. The first through fourth values may be reference values for determining the severity level of the patient (and the patient state set according to the severity level). The first through fourth values may be determined according to the specific values set as labels.

Furthermore, the first through fourth values may also be set as the average value of first score values from a plurality of other patients having physiological information similar to that of the patient. In particular, the processor 110 may set the fifth through ninth values by adjusting the same with the average value of the first scores corresponding to the normal state of the patient determined based on physiological data acquired from the patient in the past. That is, the processor 110 may repeatedly acquire the first score in the normal state of the patient from the first neural network model 30 based on physiological data repeatedly acquired from the patient in the past, and may set the reference values, i.e., the first through fourth values, by adjusting the fifth through ninth values with the average value of the repeatedly acquired first scores. As one example, the processor 110 may primarily adjust the fifth through ninth values with the average first score values of a plurality of other patients having physiological information similar to that of the patient, and thereafter secondarily adjust the primarily adjusted fifth through ninth values with the average value of the acquired first scores to set reference values (first through fourth values) suitable for the user. Thereafter, the processor 110 may set the first through fourth values within appropriate ranges including the adjusted fifth through ninth values.

Also, according to an embodiment of the present disclosure, the processor 110 may analyze physiological data based on a neural network model trained to determine whether a patient has a disease, acquire a score for the likelihood of the disease in the patient, and predict the disease in the patient based on the acquired score. The higher the acquired score, the processor 110 may determine that the likelihood of the disease occurring in the patient is high or may predict that the disease has occurred. Hereinafter, a neural network model trained to determine whether the patient has a disease is referred to as a second neural network model, and a score acquired from the second neural network model is referred to as a second score.

The disease in the patient includes various diseases such as heart failure, hypertension, and left ventricular systolic dysfunction, and the second neural network model may be trained, based on the labeled disease types in the training data, to determine the presence or absence of a specific disease.

The processor 110 may analyze the ECG data using the second neural network model trained based on training data including a plurality of physiological data (i.e., ECG data). The processor 110 may extract feature information related to a specific disease from the ECG data using the second neural network model and determine the likelihood of the disease in the patient based on the extracted feature information.

To this end, the processor 110 may acquire training data including input data composed of a plurality of ECG data acquired from different patients and label data to which labels indicating the presence of a specific disease (e.g., left ventricular systolic dysfunction) of each patient are assigned, and may pre-train the second neural network model using the acquired training data. The training data may include feature information (e.g., P-wave, QRS complex, T-wave) extracted from an ECG signal (more specifically, an ECG signal corresponding to the ECG data). The processor 110 may train the second neural network model to determine the likelihood of a specific disease in the patient based on such feature information. The processor 110 may input the training data to the second neural network model and calculate a loss function based on a difference between an output value of the second neural network model in the training process and the label data. The loss function may be defined as a cross-entropy loss or an objective function that optimizes a balance between precision and recall. Based on the calculated loss function, the processor 110 may adjust weights of the model through backpropagation. By repeating this process, the processor 110 may improve the classification performance of the second neural network model with respect to a specific disease, and may finally acquire the second neural network model trained to determine whether the patient has a specific disease based on the ECG data and to calculate a score (or probability value) relating thereto.

Meanwhile, the ECG signals corresponding to the ECG data included in the training data used for training the second neural network model may commonly have a preset length. In this regard, the embodiment of the present disclosure described above may be commonly applied.

Meanwhile, the second neural network model may be implemented as a multi-layer perceptron (MLP), a convolutional neural network (CNN), a recurrent neural network (RNN), a generative adversarial network (GAN), or the like.

According to an embodiment of the present disclosure, the processor 110 may input the acquired ECG data to the pre-trained second neural network model to acquire a score relating to a specific disease in the patient. For example, the processor 110 may input the ECG data acquired from the patient to the pre-trained neural network model to acquire a score (hereinafter referred to as a second score) relating to the likelihood of heart failure in the patient. The processor 110 may determine the likelihood of a specific disease in the patient based on the acquired score. In particular, when the acquired second score is determined to be equal to or greater than a reference value, the processor 110 may determine that heart failure has occurred in the patient. On the other hand, when the acquired score is less than the reference value, the processor 110 may determine that heart failure has not occurred in the patient, that is, the patient is in a normal state.

The reference value, which is a criterion for determining whether a specific disease, for example, heart failure, has occurred, may be set differently for each patient. To this end, the processor 110 may repeatedly acquire the ECG data for the patient, and may set the reference value by analyzing the acquired ECG data and reflecting the patient's constitution, health state, age, sex, and the like. In particular, the processor 110 may input the ECG data acquired from the patient in a normal state to the pre-trained second neural network model to acquire a second score, and may set the reference value for determining whether heart failure has occurred based on the acquired second score. For example, the processor 110 may repeatedly acquire ECG data from a wearable device (e.g., a smart watch, a smart ring) worn by the patient, and may set the reference value considering the patient's health state by analyzing the ECG of the patient in a normal state based on the acquired ECG data. Meanwhile, it goes without saying that the computing device 100 may be implemented as the above-described wearable device.

According to an embodiment of the present disclosure, the processor 110 may input the acquired ECG data and physiological information of the patient to the pre-trained second neural network model to acquire a score relating to a specific disease in the patient. The physiological information may include at least one of the patient's age, sex, height, and weight. To this end, the second neural network model may include a fully connected layer to which the physiological information of the patient is input. To this end, physiological information of the patient from whom each ECG data was acquired may be included together in the training data.

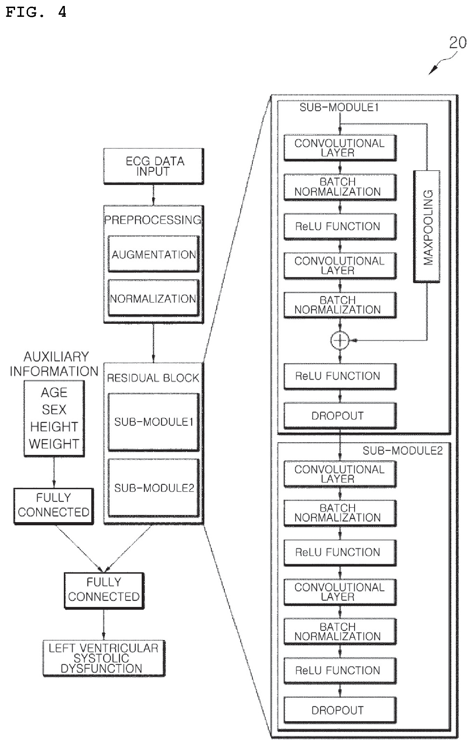

FIG. 4 is a block diagram of a second neural network model according to an embodiment of the present disclosure.

Referring to FIG. 4, the second neural network model may include a neural network composed of at least one residual block to which ECG data is input. The residual block may include a plurality of sub-modules, and each sub-module may include a plurality of convolutional neural network (CNN) layers, a batch normalization layer, and a ReLU activation function layer, and may include a dropout layer. The first sub-module illustrated in FIG. 4 may include a max pooling layer that directly inputs normalized ECG data to the last ReLU activation function layer. Also, the second neural network model may include a fully connected layer to which auxiliary information such as age, sex, height, and weight is input. The output of the fully connected layer and the output of the residual block may be concatenated to derive the probability of onset of peripartum cardiomyopathy. ECG data input to the first neural network model 30 may be preprocessed by down-sampling and noise-applied augmentation before being input to the residual block. For example, the sampling rate of the ECG data was down-sampled from 500 Hz to 250 Hz, and data augmentation with various noise applied was used.

Meanwhile, the second neural network model may include a plurality of neural networks corresponding to the number of leads used to acquire the ECG data. That is, the second neural network model may include individual neural networks to which ECG data measured by each lead is respectively input. For example, the second neural network model may include a first sub-neural network model of the second neural network model trained based on ECG data measured with 12 multiple leads. Also, the first neural network model 30 may further include a second sub-neural network model of the second neural network model trained based on ECG data measured with 6 limb leads or 6 precordial leads. Also, the first neural network model 30 may further include a third sub-neural network model of the second neural network model trained based on ECG data measured with a single lead.

The second neural network model may selectively use at least one of the first sub-neural network model of the second neural network model, the second sub-neural network model of the second neural network model, or the third sub-neural network model of the second neural network model depending on the number of leads used to acquire the ECG data. The first through third sub-neural network models of the second neural network model may be trained with training data composed of ECG data corresponding to the measurement method (i.e., the number of leads) used to acquire each ECG data.

Also, the second neural network model may include a plurality of sub-neural network models depending on disease type. For example, the second neural network model may include at least one of a sub-neural network model that determines whether the patient has heart failure based on the ECG data (a fourth sub-neural network model of the second neural network model), a sub-neural network model that determines whether the patient has left ventricular systolic dysfunction (a fifth sub-neural network model of the second neural network model), and a sub-neural network model that determines whether the patient has hypertension (a sixth sub-neural network model of the second neural network model).

Each of the sub-neural network models (the fourth through sixth sub-neural network models of the second neural network model) may be pre-trained with training data composed of label data corresponding to each disease type. In this regard, the description given above may be applied equally, and a detailed description thereof is omitted.

Meanwhile, the processor 110 may determine the severity level of the patient according to the disease based on the first and second scores. That is, the processor 110 may determine that the severity level is manifested according to the disease identified based on the second score. In particular, the processor 110 may respectively acquire second scores from sub-neural network models corresponding to each disease type, and may determine the disease that is the cause of the severity level of the patient based on the acquired plurality of second scores.

The processor 110 may determine the relevance between the disease and the disease type based on the first score, the second score, and the disease type corresponding to the second score. In particular, the processor 110 may determine that the higher the second score, the more the disease has affected the severity level of the patient, and thus the degree of relevance is high. The processor may determine the relevance between the second score and the first score in consideration of the disease type. That is, even for the same second score, the relevance to the severity level may be determined differently depending on the disease type. To this end, the processor may input the first and second scores and disease type information to a pre-trained neural network model (hereinafter, a third neural network model) to calculate an index indicating the relevance. However, without being limited thereto, the processor 110 may also acquire the relevance index based on a machine learning model (e.g., a logistic regression model), a pre-generated table (e.g., a look-up table) relating to the relevance of the first and second scores and the disease type information.

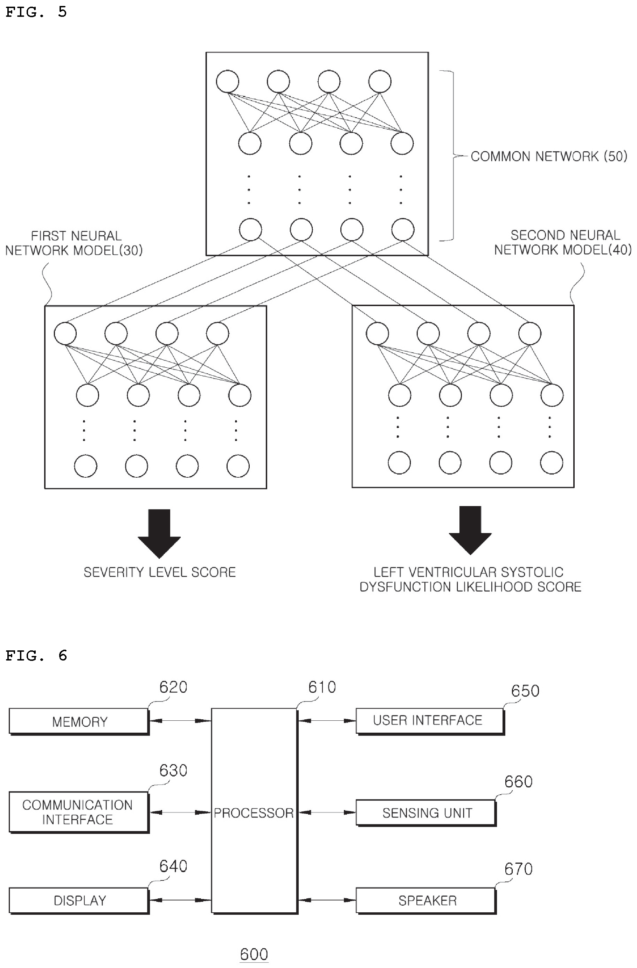

FIG. 5 is an exemplary diagram of a first neural network model 30 and a second neural network model according to an embodiment of the present disclosure.

To this end, according to an embodiment of the present disclosure, a network that extracts feature information corresponding to the patient state from physiological data may be shared. For example, the first neural network model 30 and the second neural network model may share a portion (a partial neural network) of the neural network model that extracts feature information, and may each include a classifier that classifies the severity level of the patient and the degree of disease likelihood based on the extracted feature information. For example, referring to FIG. 5, the first neural network model 30 and the second neural network model 40 may share a neural network 50 that extracts feature information from the ECG data. It may be determined that the cause of the severity level determined from the first score output from the first neural network model 30 is the disease identified based on the second score output from the second neural network model 30. Meanwhile, the first neural network model 30 may include a plurality of sub-neural network models trained to determine the severity level related to each disease type, and each sub-neural network model may share a neural network that extracts feature information with sub-neural network models corresponding to each disease type (e.g., fourth through sixth sub-neural network models of the second neural network model), respectively. The processor may determine the disease type having the highest score among the plurality of second scores acquired from the plurality of second sub-neural network models, and may determine the severity level corresponding to the determined disease type and the disease affecting the severity level as the corresponding disease type. However, weights corresponding to each disease type may be applied to the plurality of second scores, and the disease type having the highest score may be determined.

Also, according to an embodiment of the present disclosure, the processor 110 may determine a state of the patient by classifying the time before cardiac arrest into a plurality of time units together with the severity level of the patient. For example, the processor 110 may use a fourth neural network model to determine the state of the patient as a state within a first time period before cardiac arrest, a state within a second time period before cardiac arrest, a state within a third time period before cardiac arrest, a state within a fourth time period before cardiac arrest, or a state within a fifth time period before cardiac arrest. To this end, the processor 110 may train the fourth neural network model with training data composed of ECG data acquired from a plurality of patients corresponding to each time unit. The training method of the fourth neural network model may be equally applied from the description of the present disclosure described above. The fourth neural network model may be particularly trained by extracting only feature information related to cardiac arrest from the ECG data. This is because, unlike the first neural network model 30, which relates to the severity level of the patient, the fourth neural network model focuses solely on predicting the cardiac arrest, i.e., death, of the patient.

The remaining time until cardiac arrest and whether cardiac arrest will occur in the patient based on the fourth neural network model may be performed for a patient having a severity level equal to or greater than an intensive care unit hospitalization state with a high severity level. That is, when the severity level of the patient is determined to be equal to or greater than the intensive care unit hospitalization state based on the first score, the processor 110 may monitor the patient's state using the fourth neural network model. Meanwhile, the processor 110 may determine and manage the likelihood of cardiac arrest in the patient and within how many hours before cardiac arrest the patient is based on a score (a third score) acquired from the fourth neural network model. The reference values applied to the third score may be equally applied from the content regarding the first score, and a detailed description thereof is omitted.

Meanwhile, the fourth neural network model may be implemented as a multi-layer perceptron (MLP), a convolutional neural network (CNN), a recurrent neural network (RNN), a generative adversarial network (GAN), or the like.

FIG. 6 is a detailed configuration diagram of a computing device 600 according to another embodiment of the present disclosure.

Referring to FIG. 6, the computing device 600 according to an embodiment of the present disclosure includes a processor 610, a memory 620, a communication interface 630, a display 640, a user interface 650, a sensing unit 660, and a speaker 670. Among the configurations illustrated in FIG. 6, a detailed description of configurations overlapping with those illustrated in FIG. 1 is omitted.

The display 640 may display various images. The images include both still images and moving images. The display 640 may also output an ECG signal, and may also output a noise score, a noise level, and the like of the ECG signal. The display 640 may be implemented as various types of displays such as a liquid crystal display (LCD), organic light emitting diodes (OLED), liquid crystal on silicon (LCoS), and digital light processing (DLP). Also, within the display 640, a driving circuit, a backlight unit, and the like that may be implemented in forms such as amorphous silicon (a-Si) TFT, low temperature poly silicon (LTPS) TFT, and organic TFT (OTFT) also may be included. Meanwhile, the display 640 may be implemented as a touch screen by being combined with a touch panel, and in this case, the display 640 may perform a function of an input interface that receives touch input of a user as well as an output interface that outputs images through the touch screen. The display 640 may display the severity level of the patient, the first score, the second score, and the like.

The user interface 650 is a configuration used for the computing device 600 to perform interaction with a user, and may include at least one of a touch sensor, a motion sensor, a button, a jog dial, and a switch, but is not limited thereto.

The sensing unit 660 senses a physiological signal of a patient to acquire physiological data. As one example, the sensing unit 660 may detect an electrical signal of the heartbeat of the patient through at least one electrode included in the sensing unit 660 to acquire the ECG data of the patient.

The speaker 680 is a component that outputs various audio data on which various processing tasks such as decoding, amplification, and noise filtering have been performed by an audio processing unit (not shown). The speaker 680 may output various notification sounds or voice messages. According to an embodiment of the present disclosure, the processor 910 may convert an electrical signal into a user's (1) voice and output it via the speaker 680. As one example, the speaker 680 may output a voice message notifying the severity level of the patient, the remaining time until cardiac arrest, and an emergency state of the patient.

Meanwhile, according to an embodiment of the present disclosure, a non-transitory computer-readable medium storing a program for performing the above-described method for determining a severity level of a patient based on artificial intelligence may be provided. The non-transitory computer-readable medium refers to a medium that stores data semi-permanently and is readable by a device, rather than a medium that stores data for a short moment, such as a register, a cache, or a memory. Specifically, the above-described various applications or programs may be stored in and provided on a non-transitory computer-readable medium such as a CD, a DVD, a hard disk, a Blu-ray disc, a USB, a memory card, or a ROM.

The various embodiments of the present disclosure described above may be combined with additional embodiments, and may be modified within the scope understandable by a person of ordinary skill in the art in light of the foregoing detailed description. The embodiments of the present disclosure are intended to be illustrative in all aspects and not restrictive. For example, each component described as a single unit may be implemented in a distributed manner, and likewise, components described as distributed may be implemented in a combined form. Therefore, all changes or modified forms derived from the meaning and scope of the claims of the present disclosure and their equivalent concepts are to be interpreted as being included in the scope of the present disclosure.

## Claims

1. A method for determining a severity level of a patient based on a deep learning model, performed by a computing device including at least one processor, the method comprising:
acquiring physiological data of the patient; and
determining the severity level of the patient by analyzing the physiological data based on a pre-trained first neural network model,
wherein the pre-trained first neural network model is trained based on training data to which labels corresponding to patient states and treatment methods classified according to patient severity levels are assigned.

2. The method of claim 1, wherein
the labels corresponding to patient states and treatment methods classified according to patient severity levels are
classified into a patient in a normal state, a patient receiving outpatient treatment, a patient hospitalized in a general ward, a patient hospitalized in an intensive care unit, and a patient within a preset time period before cardiac arrest.

3. The method of claim 2, wherein
the determining of the severity level of the patient comprises
inputting the physiological data into the pre-trained first neural network model to extract feature information corresponding to a state of the patient, acquiring a first score corresponding to the severity level of the patient based on the extracted feature information, and determining the severity level of the patient based on the acquired first score.

4. The method of claim 3, wherein
the determining of the severity level of the patient based on the acquired first score comprises
determining the severity level of the patient as a normal state when the acquired first score is less than a first value, determining the severity level of the patient as a severity level amenable to outpatient treatment when the acquired first score is equal to or greater than the first value and less than a second value, determining the severity level of the patient as a severity level requiring hospitalization when the acquired first score is equal to or greater than the second value and less than a third value, determining the severity level of the patient as a severity level requiring intensive care when the acquired first score is equal to or greater than the third value and less than a fourth value, and determining the severity level of the patient as a state within a preset time period before cardiac arrest when the acquired first score is equal to or greater than the fourth value.

5. The method of claim 4, wherein
the labels corresponding to patient states and treatment methods classified according to patient severity levels comprise:
a fifth value corresponding to the patient in a normal state, a sixth value corresponding to the patient receiving outpatient treatment, a seventh value corresponding to the patient hospitalized in a general ward, an eighth value corresponding to the patient hospitalized in an intensive care unit, and a ninth value corresponding to the patient within a preset time period before cardiac arrest.

6. The method of claim 5, wherein
the first through fourth values are
set by adjusting the fifth through ninth values with the average value of first scores corresponding to the normal state of the patient determined based on physiological data acquired from the patient in the past.

7. The method of claim 3, comprising,
by analyzing the physiological data based on a pre-trained second neural network model to acquire a second score for likelihood of a disease in the patient, determining the severity level of the patient according to the disease based on the first and second scores.

8. The method of claim 7, wherein
the determining of the severity level of the patient according to the disease comprises:
determining the severity level of the patient according to the disease by determining relevance between the disease and the severity level of the patient based on the first score, the second score, and disease type information of the patient.

9. The method of claim 7, wherein
the pre-trained first and second neural network models
share a network extracting, from the physiological data, feature information corresponding to the state of the patient.

10. A computing device for determining a severity level of a patient based on a deep learning model, the computing device comprising:
a processor comprising at least one core;
a memory storing program code executable by the processor; and
a communication interface,
wherein the processor is configured to:
acquire physiological data of the patient; and
determine the severity level of the patient by analyzing the physiological data based on a pre-trained first neural network model,
wherein the pre-trained first neural network model is trained based on training data to which labels corresponding to patient states and treatment methods classified according to patient severity levels are assigned.

11. A computer program stored on a computer-readable storage medium, the computer program, when executed by one or more processors, causing operations for determining a severity level of a patient based on a deep learning model to be performed,
the operations comprising:
acquiring physiological data of the patient; and
determining the severity level of the patient by analyzing the physiological data based on a pre-trained first neural network model,
wherein the pre-trained first neural network model is trained based on training data to which labels corresponding to patient states and treatment methods classified according to patient severity levels are assigned.
